# EUROPEAN PATENT APPLICATION

(11) **EP 0 875 144 A1**
(43) Date of publication of application: **04.11.1998**
(21) Application number: 98302966.1
(22) Date of filing: 16.04.1998
(51) Int. Cl.: A01N 25/34, A01N 25/10

(54) **Antimicrobial and anti-algae agent for plants, a plant-growing structure and a method for suppressing propagation of fungi and algae in plant-growing**

(30) Priority: 17.04.1997 JP 114170/97; 26.12.1997 JP 366581/97
(71) Applicant: HAYAKAWA RUBBER COMPANY LIMITED, Fukuyama City, Hiroshima Pref. (JP); Sanyo Turf Co., Ltd., Jinseki-Gun, Hiroshima Pref. (JP)
(72) Inventor: Urakami, Kazuto, Fukuyama City, Hiroshima Pref. (JP); Hirokawa, Kazuya, Fukuyama City, Hiroshima Pref. (JP); Miyachi, Yosifumi, Jinseki-Gun, Hiroshima Pref. (JP)
(74) Representative: Daniels, Jeffrey Nicholas

(57) **Abstract**

An antimicrobial and anti-algae agent for plants, includes chips and/or pieces made of rubber or plastic. When this antimicrobial and anti-algae agent is applied around a plant, the growth of the fungi and the algae can be suppressed or controlled. A plant-growing structure comprising a plant, a cultivating medium at which said plant grows, and the antimicrobial and anti-algae agent is also disclosed.

## Description

The present invention relates to an antimicrobial and anti-algae agent for plants. Further, the invention relates to a plant-growing structure in which such an antimicrobial and anti-algae agent is applied to a plant as well as a method for suppressing propagation of fungi and algae in growing a plant by applying the anti-microbial and anti-algae agent to the plant. In particular, the invention is to control diseases in turfgrass or plants in golf fields, athletic fields, or plant pots and to suppress propagation of fungi and algae there.

Diseases in turfgrass have been treated by spraying a chemical upon the turfgrass when the diseases occurred. In a worse case, the diseases have been coped with by transplanting the turfgrass. With respect to algae, there has been no particular controlling countermeasure, and it has been a common practice that the occurrence of the algae is controlled by drying the turfgrass as much as possible. Furthermore, with respect to ornamental plants, diseases occurs at houses. In those cases, the diseases may be cured by spraying the chemical upon the plants as in the turfgrass, but it is an actual situation that the plants are let to die in most cases as they are.

The methods conventionally used for controlling the disease damages against the turfgrass are broadly classified into the following:
(1) Chemical method: treatment and prevention of disease damages upon turfgrass with chemicals
(2) Physical method: sanitizing of turfgrass with heat, light or the like, renovation of turfgrass and removal of organic residues
(3) Biological method: utilization of biological agrochemical or organic compound
(4) Cultural method: aeration, water-permeability improvement

As pathogenic fungi in turfgrass, *Fusarium spp*., *Pythium sp*., *Rhizoctonia spp*. and *Sclerotinia homococarpa Bennett* are typically famous. In order to control diseases of turfgrass with these pathogenic fungi, a chemical method is a main trend, which method sprays a chemical suitable for the prevention of these fungi upon the turfgrass.

According to such a conventional chemical-spraying method, a chemical is diluted, and sprayed onto the turfgrass by a spray or like. Further, the sprayed chemical is flown off or out or scattered if it rains or blows or if a person passes on the sprayed turfgrass. For this reason, since the effect in spraying the chemical is very small. It is time-consuming to dilute the chemical or the chemical may be scattered with wind formed in spraying. Thus, there are various problems. Such a method causes a pollution problem in some areas.

As mentioned above, there are various problems in coping with the plant diseases with the chemicals as conventionally employed. There is a limit in use of the chemicals due to the restriction upon the use amounts of the chemicals, chemical damages, etc. Furthermore, use of the chemical may cause damages upon human beings, animals, etc., and therefore should be avoided as much as possible.

However, since a certain chemical actually exhibits an enormous effect, such a chemical needs to be relied upon to control the disease damage of the plant. In such a case, use of a chemical having a special antimicrobial function may be considered. However, the product having this special antimicrobial function is costly, so that it is an actual situation that the product is difficult to be used in a wide area.

The present invention is to solve the above-mentioned problems possessed by the conventional chemicals, and to provide a method for controlling disease damages of plants without spraying the chemicals directly upon the plants.

The present invention relates to an antimicrobial and anti-algae agent for plants, said agent composed of chips and/or pieces made of rubber or plastic and adapted to control propagation of fungi and algae when the antimicrobial and anti-algae agent is applied to a circumstance of a plant.

It is time-consuming to control disease damages of turfgrass by spraying a chemical upon the turfgrass because of necessity of dilution and spraying a plant-growing structure and a method for suppressing propagation of fungi and algae in plant-growing, so that it is difficult to spray an appropriate amount of the chemical over a desired area. Further, the sprayed chemical easily flows out upon exposure to wind and rain, which causes environmental pollution. In view of this, the present inventors investigated substituting countermeasure for the chemical spraying.

Surprisingly, the present inventors discovered that chips and/or pieces made of rubber or plastic exhibit antimicrobial effect and anti-algae effect for turfgrass. Furthermore, the present inventors made strenuous investigations and reached a conclusion that the chips and/or pieces themselves no doubt suppress propagation of the fungi and the algae and last their antimicrobial effect and anti-algae effect.

According to the antimicrobial and anti-algae agent of the present invention, the antimicrobial and anti-algae effects can be exhibited without addition of a special antimicrobial agent or fungicide. If the plant antimicrobial and anti-algae agent according to the present invention is applied to be directly contacted with soil as a plant-growing medium, the propagation of the fungi and the algae can be largely suppressed.

The antimicrobial and anti-algae agent according to the present invention does not require a troublesome operation as in the conventional chemical-spraying method. When the antimicrobial and anti-algae agent is applied to be directly contacted with the plant-growing medium, the disease damage of the turfgrass can be effectively controlled. According to the present invention, the effect of chemically controlling the disease damage of the plant can be relatively easily exhibited, and this effect can be lasted over a prolonged time period.

These and other objects, features and advantages of the invention will be appreciated upon reading the following description of the invention when taken in conjunction with the attached drawings, with the understanding that some modifications, variations and changes of the same could be easily made by the skilled person in the art to which the invention pertains.

The present invention will now be described by way of example only with reference to the accompanying drawings, in which:-
Fig. 1 is a vertically sectional view for illustrating a state in which the antimicrobial and anti-algae agent according to the present invention is broadcasted;
Fig. 2 is a photograph of a turf in which a treated area is compared with a non-treated area;
Fig. 3 is a schematic diagram for explaining Fig. 2;
Fig. 4 is a photograph of an algae-propagating state;
Fig. 5 is a schematic diagram for explaining Fig. 4;
Fig. 6 is an enlarged photograph of a boundary zone between the treated area and the non-treated area;
Fig. 7 is a schematic diagram for explaining Fig. 6;
Fig. 8 is a photograph of a green area before treatment;
Fig. 9 is a schematic diagram for illustrating Fig. 8;
Fig. 10 is a photograph of the green area after the treatment;
Fig. 11 is a schematic diagram for explaining Fig. 10;
Fig. 12 is a photograph of a green area in which a treated area is compared with a non-treated area;
Fig. 13 is a photograph of the treated area;
Fig. 14 is a photograph of the non-treated area; and
Fig. 15 is a graph for showing the growing degree of pathogenic fungi.

The plant antimicrobial and anti-algae agent according to the present invention can be produced by using a material such as rubber or plastic to be usually employed in the rubber and plastic industries. The plant antimicrobial and anti-algae agent according to the present invention preferably includes at least one kind of an ingredient selected from the group consisting of zinc oxide, stearic acid, an activating agent, a processing aid, filler, vulcanization accelerator, resin, viscosity aid, tackifier aid, and sulfur. The following compounding rates of the above ingredients are preferred.

| | |
|---|---|
| Rubber and/or polymer | 100 parts by weight |
| ZnO | 2 to 7 parts by weight |
| Stearic acid | 0.5 to 2 parts by weight |
| Activating agent | 0.3 to 5 parts by weight |
| Filler | 10 to 800 parts by weight |
| Process oil | 5 to 50 parts by weight |
| Pigment | 1 to 10 parts by weight |
| Vulcanization accelerator | 0.5 to 5 parts by weight |
| Sulfur | 0.1 to 7 parts by weight |

As the above-mentioned ingredients, those ordinarily used in the rubber or polymer industries such as the tire industry may be employed. The chips and/or pieces containing any one of these ingredients exhibits antimicrobial and anti-algae effect. In addition to the above preferred ingredients, a surface active agent may be added. This surface active agent functions to control flow-out of the plant antimicrobial and anti-algae agent according to the present invention from the surface of the soil with rain water or sprinkled water.

The plant antimicrobial and anti-algae agent according to the present invention is made of chips and/or pieces which can be shaped in various forms from various materials. The plant antimicrobial and anti-algae agent according to the present invention comprises such chips and/or pieces as to cause no trouble in use for plants such as turfgrass. The chips and/or pieces may be ground pieces of vulcanized rubber, pieces obtained by hot cutting plastic, pieces obtained by extruding a molding material and cutting the resulting molded product. The chips and/or pieces may be either of fixed forms or of non-fixed forms. The size, the broadcasting amount and density of the chips and/or pieces and/or pieces may be appropriately selected depending upon the broadcasting location (height of plant such as turfgrass, growing density thereof, etc.). As to the putting green, the volume of each of the chips and/or pieces is not more than 16 mm³.

Each of the chips and/or pieces of the plant antimicrobial and anti-algae agent according to the present invention has preferably a volume of 0.00005 to 3.0 cm³, preferably 0.0001 to 1.0 cm³, and preferably has a specific density of 0.5 to 4.0, preferably 0.5 to 2.8. The above volume range is set in view of the fact that the chips and/or pieces are favorably positioned around the plant such as turfgrass. The larger the specific gravity, the more effectively is the flow-out of the plant antimicrobial and anti-algae agent controlled. The reason why the specific gravity is preferably 0.5 to 4.0 is that 0.5 is set in view of the water plant cultivation, whereas 4.0 is set because the chips and/or pieces are likely to enter the land in this case, if the specific gravity exceeds 4.0.

Rubber and plastic to be used in the plant antimicrobial and anti-algae particles of the present invention include rubber alone, plastic alone, a mixture of rubber and plastic, various rubber mixtures, and various plastic mixtures. The rubber includes natural rubber and synthetic rubber (styrene-butadiene rubber, butadiene rubber, chloroprene rubber, isoprene rubber, ethylene-propylene rubber, etc.), and the plastic includes synthetic resins such as polystyrene, polyethylene, polyvinyl chloride and polyethylene terephthalate and natural resins.

The plant to be aimed at by the present invention includes flowers, turfgrass, ornamental plants that people ordinarily cultivate as well as their seeds and seedlings. The turfgrass includes Zoysia spp. as well as western turfgrass. That is, the turfgrass includes both warm-season turfgrass species and cool-season turfgrass species. The medium at which the plant grows includes soil and soil with a water layer on its surface. The soil includes natural soil, artificial soil, and various culture media. The fungi and the algae is a class generally encompassing the fungi and the algae, and includes pathogenic fungi such as Fusarium fungi, Pythium spp., Rhizoctonia spp. and Sclerotinia homoeocarpa Bennett and algae such.

The plant antimicrobial and anti-algae particles according to the present invention preferably contain at least one of an antimicrobial agent and an anti-algae agent to control the disease damages upon the plant such as turfgrass. Because, such an antimicrobial agent and a bactericide can afford a desired disease damage-controlling effect upon the plant antimicrobial and anti-algae particles.

The plant antimicrobial and anti-algae particles according to the present invention are preferably produced from a polymer material obtained by preliminarily adding, mixing and kneading at least one of an antimicrobial agent and a bactericide into rubber or plastic material. The plant antimicrobial and anti-algae particles in which an antimicrobial agent and/or a bactericide is kneaded into rubber or plastic material can be easily formed, and their disease damage controlling effect can be lasted with a small flow-out amount of the antimicrobial agent and/or an anti-algae agent.

It is preferable that the plant antimicrobial and anti-algae particle according to the present invention includes a particle body and a coating layer placed around the particle body, and this coating layer contains at least one of an antimicrobial agent and an anti-algae agent. Such a particle can be produced by coating an appropriate particle body with a coating agent into which the antimicrobial agent and/or the anti-algae agent is added and mixed.

The antimicrobial agent is an agent to control propagation of fungi or occurrence of fungi. According to the present invention, a material containing a metallic ingredient such as mercury, silver or copper which has oligodynamic function can be used as the antimicrobial agent. The bactericide is an agent to kill fungi occurring. Ordinary grass type registered turfgrass bactericides (such as Captan or Thiuram) may be used in the present invention. The present invention is not limited particularly to the above chemicals, chemicals containing natural extract such as Japanese cypress thiol (hinokitiol) may be also used as in the above chemicals.

The addition amount of the antimicrobial agent and/or the bactericide may be set at a desired range depending upon the kind of the chemical and the degree of the disease damage upon the turfgrass. For example, if a chemical in which silver ions are contained in a zeolite structure (trade name: Zeomix manufactured by Sinanen Co., Ltd. is used, the addition amount of the chemical to the particle is preferably 0.1 to 2 wt% with respect to the weight of the chips and/or pieces. Even if the addition amount is more than 2 wt%, an effect meeting the addition amount cannot be obtained. The antimicrobial agent is incorporated into the particle to control the propagation of the disease damage, and a similar effect to that in case of the incorporation of the fungicide is recognized.

The plant antimicrobial and anti-algae particles according to the present invention can exhibit the antimicrobial and anti-algae effect at a stationary location for a long time without being limited to a particular location for the particles to be arranged or applied. Further, the antimicrobial agent or the bactericide incorporated into the plant antimicrobial and anti-alga particles according to the present invention is not flown out or scattered with water, wind or the like.

### (Examples)

In the following, the present invention will be more concretely explained by referring to specific examples.

### Example 1

Starting materials in a compounding recipe shown in Table 1 were kneaded with a kneader to be ordinarily used in the rubber industry, and vulcanized in a vulcanizer also usable in the rubber industry. Then, rubber powder was obtained by grinding the resulting rubber composition. Thereafter, the rubber powder was sieved through a JIS sieve, thereby obtaining rubber chips and/or pieces having 10 to 14 meshes (about 0.83 to 2.4 mm³ when measured assuming that the chip are spherical). The rubber chips and/or pieces were adjusted to a true specific gravity of 2.3, and colored green to give quasi-green.

**Table 1**

| Compounding ingredient | parts by weight |
|---|---|
| Ethylene propylene rubber | 140 |
| Zinc oxide | 10 |
| Stearic acid | 2 |
| Activating agent (polyethylene glycol) | 4 |
| Processing aid (Cumarone-indene resin) | 10 |
| Filler (Barium sulfate) | 800 |
| Process oil (Paraffinic oil) | 40 |
| Pigment (green LX*) | 5 |

| Vulcanization accelerator | |
|---|---|
| (Vulcanization accelerator M) | 1.0 |
| (Vulcanization accelerator TT) | 0.5 |
| (Vulcanization accelerator TRA) | 0.5 |
| (Vulcanization accelerator EZ) | 1.5 |
| | Total : 3.5 |
| Sulfur | 4.5 |

| | |
|---|---|
| * : Trade Name, manufactured by Sanyo Colorant Co., Ltd. | |

### (1) Turfgrass anti-algae treatment

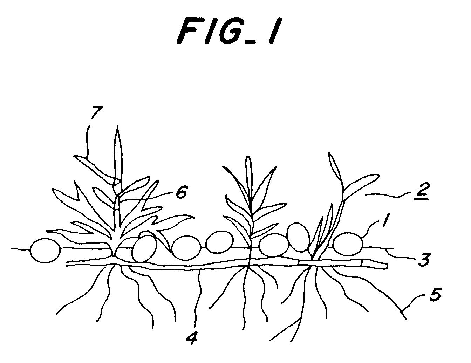

An anti-algae effect of the plant antimicrobial and anti-algae agent according to the present invention was tested. More specifically, the rubber chips and/or pieces produced in Example 1 were broadcasted at a rate of 300 g/m² on a putting green of a golf course in which algae grew. The rubber chips and/or pieces were contacted directly to the surface of soil by brushing. Fig. 1 is a vertically cross sectional view for illustrating a state in which the rubber chips and/or pieces are broadcasted on the green area. A turfgrass 2 includes erect stems 6, leaves 7, roots 5 and stolon. The rubber chips and/or pieces were broadcasted onto the ground surface 3 at which the turfgrass 2 grew.

It was visually observed one week after the broadcasting that the algae disappeared in a treated zone in which the rubber chips and/or pieces were broadcasted, whereas the algae remained in a non-treated zone in which no rubber chips and/or pieces were broadcasted.

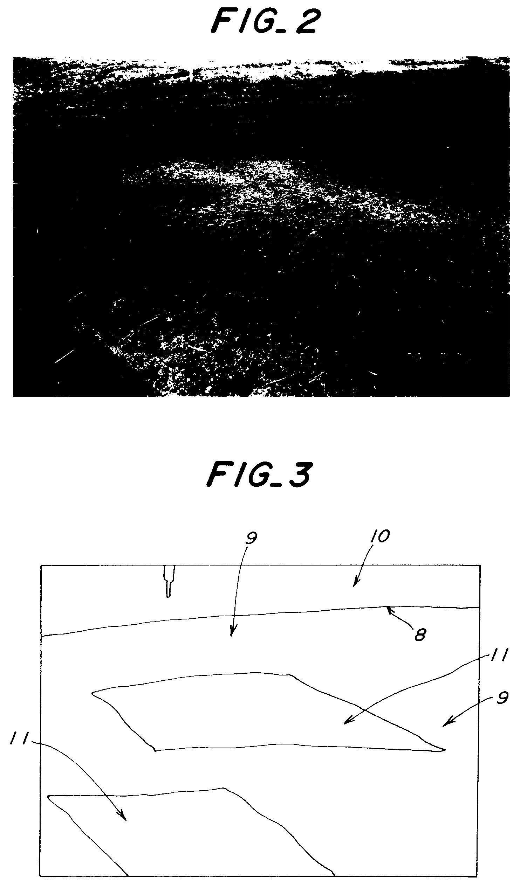

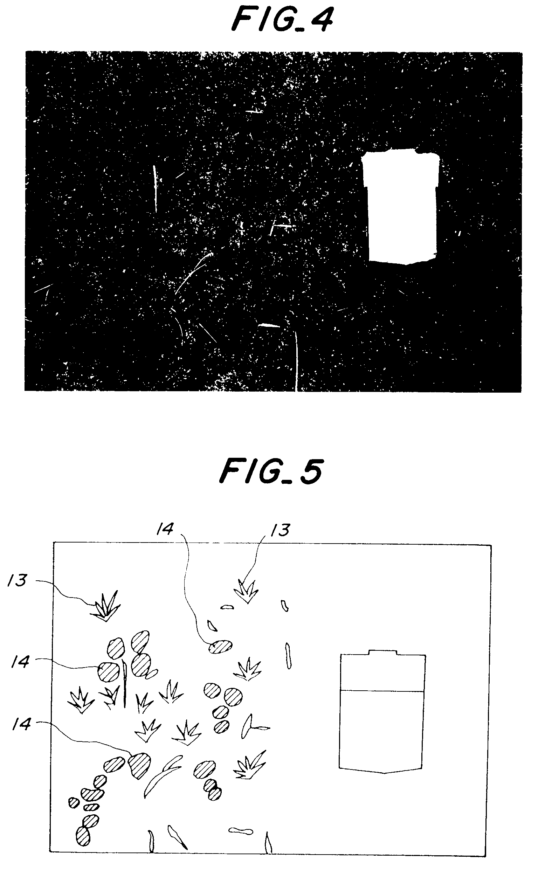

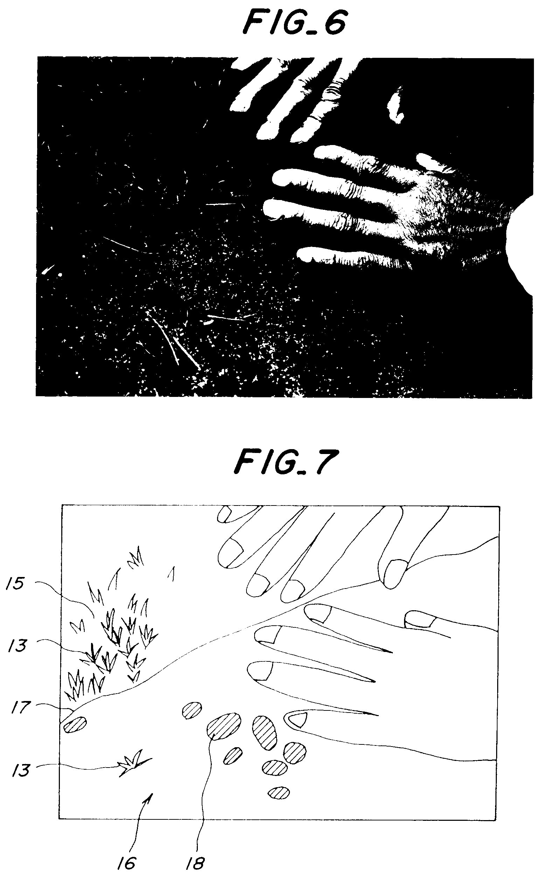

Figs. 2 to 7 show a result in the algae-controlling treatment. Fig. 2 is a photograph in which the treated zone is compared with the non-treated zone. Fig. 3 is a diagram for illustrating Fig. 2. In Fig. 3, a boundary line 8 is drawn between a green area 9 and the rough 10. The non-treated zones 11 are seen in Fig. 3, and it is seen that in the non-treated zones 11, algae grew and the growth of the turfgrass was more interrupted as compared with the surrounding treated zone 12. As shown in Fig. 2, the non-treated zone 11 look whitish. Fig. 4 is a photograph for showing a growing state of the algae, and Fig. 5 is a diagram for illustrating Fig. 4. In Figs. 4 and 5, black portions 14 distributed in a spot-like fashion among the turfgrass 13 are grown algae. Fig. 6 is an enlarged photograph of a boundary portion between the treated zone and the non-treated zone. Fig. 7 is a diagram for illustrating Fig. 6. In these figures, the upper portion and the lower portion are the treated zone 15 and the non-treated zone 16, respectively. A boundary line 17 denotes a boundary between the treated zone 15 and the non-treated zone 16. In the treated zone 15, the turfgrass 13 satisfactorily grew, whereas in the non-treated zone 16, the algae grew, the surface of the soil became black at locations 18, and the growth of the turfgrass was interrupted.

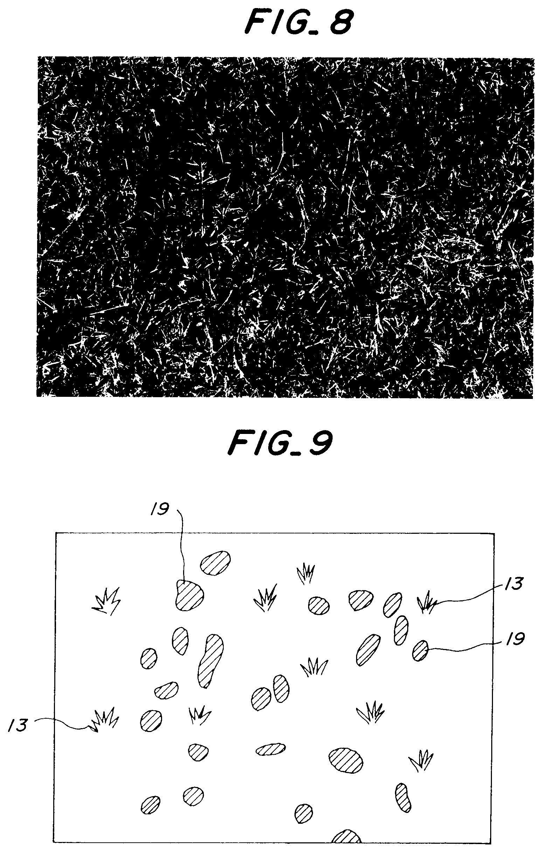

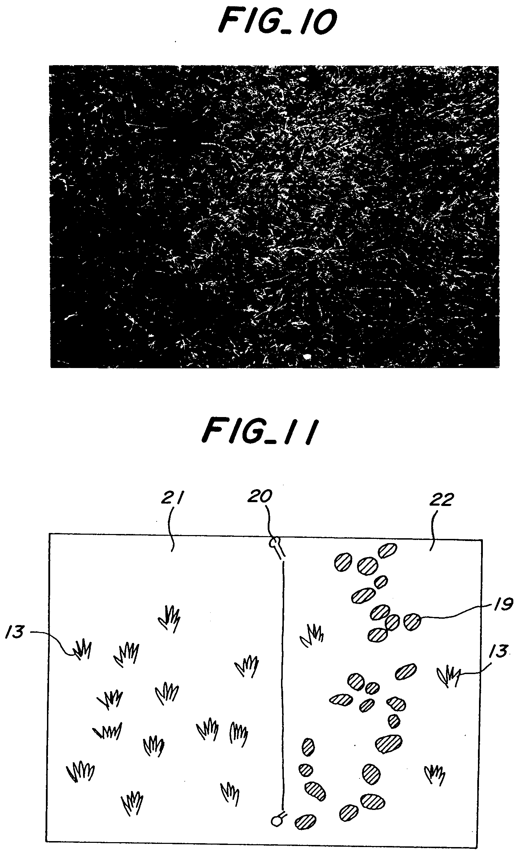

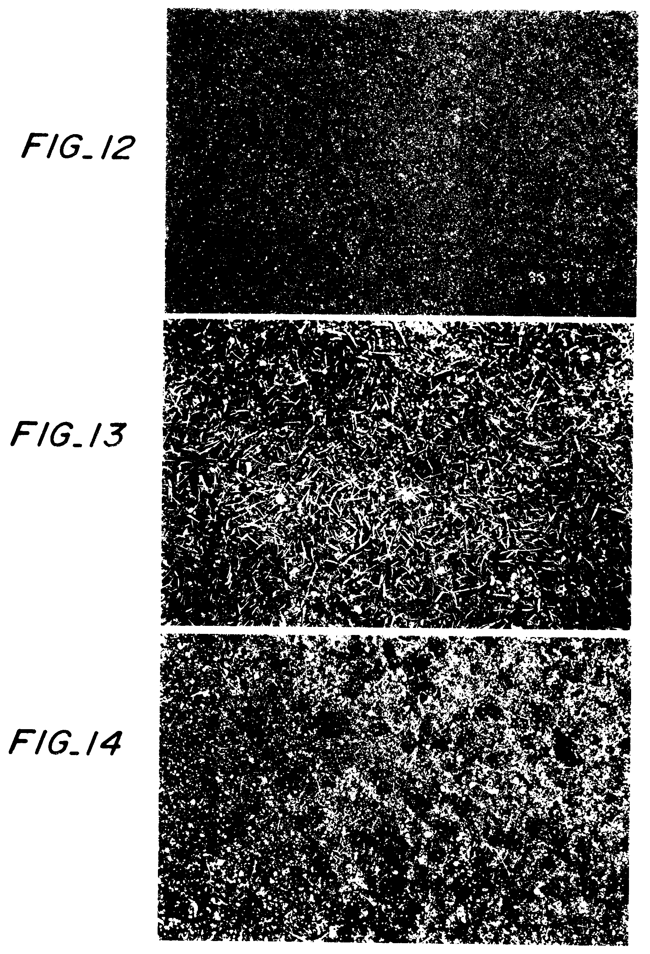

Figs. 8 to 11 show a green area before or after an algae-controlling treatment. Fig. 8 is a photograph for showing the green area before the treatment, and Fig. 9 is a diagram for illustrating Fig. 8. As shown in these figures, algae-grown portions 19 lie scattered on the ground among the turfgrass 13. Fig. 10 is a photograph for showing the green area after the treatment, and Fig. 11 is a diagram for illustrating Fig. 10. In the algae-controlling treatment, a substantially central portion of the green area shown in Fig. 8 was divided into a treated zone 21 and a non-treated zone 22 by means of poles. The rubber chips and/or pieces were sprinkled in the treated area 21 on September 17, 1997. Fig. 10 is a photograph of the treated green area taken on October 8, 1997 (21 days after the sprinkling with the rubber chips and/or pieces). As shown in these figures, the algae disappeared in the treated zone 21 and the growth of the turfgrass 13 was promoted. In the non-treated zone 22, the grown algae remained, and the growth of the turfgrass 13 was interrupted.

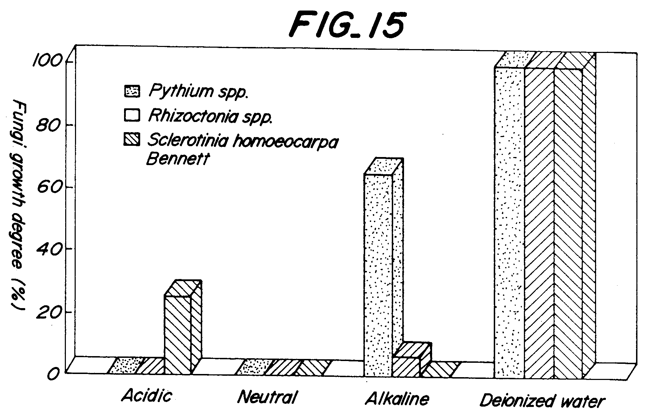

Figs. 12 to 14 are photographs taken on September 8, 1996. Fig. 12 is a photograph for showing a treated zone and a non-treated zone in parallel. In Fig. 12, the left and right portions denote the treated zone and the non-treated zone, respectively. Fig. 13 is a photograph for showing the treated zone, and Fig. 14 is a photograph for showing the non-treated zone. These photographs were taken on a location different from those in Figs. 2 to 11. As shown in the left side of Fig. 12 and Fig. 13, the algae disappeared and the growth of the turfgrass was promoted in the treated zone. On the other hand, as shown in the right side of Fig. 12 and Fig. 14, the algae did not disappear and the growth of the turfgrass was interrupted.

### (2) Turfgrass antimicrobial treatment test

Perennial ryegrass (*Lolium perenne*) was planted in a nursery having an area of 500 m², and the grass was uniformly moved at the moving height of 3.5 mm in February. The rubber chips and/or pieces in Example 1 were sprinkled to be directly contacted with the soil at a density of 300 g/m² over a half area of 250 m². Much symptom attributable to *Pythium spp*. occurred in the non-treated zone in July, whereas such a symptom occurred nowhere in the treated zone.

### (3) Antimicrobial effect test with an extract liquid of the rubber chips and/or pieces

Further, for a confirmation purpose, the rubber chips and/or pieces in Example 1 were immersed in 100 ml of each of aqueous solutions at pH 3, pH 7 and pH 12 at room temperature, thereby obtaining extracted liquids. At that time, pH buffer liquids were used as the aqueous solutions, respectively. With respect to each of the extracted liquids, a laboratory dish test was effected through cultivation with agar. As pathogenic fungi, three kinds of fungi typical to the turfgrass, i.e., *Pythium spp*., *Rhizoctonia spp*. AG2-2, and *Sclerotinia homoeocarpa Bennett*, were used. Results obtained are shown in Fig. 15.

Fig. 15 is a graph showing the degree of the growth with respect to three kinds of the disease-causing fungi. In Fig. 15, the degree of the growth with respect to deionized water was taken as 100%, and those obtained with respect to the respective extract liquids were shown relative to that for the deionized water. In the pH 3 extract liquid, the *Sclerotinia homoeocarpa Bennett* exhibited the growth at 25%, but the other two did not grow. In the pH 7 extract liquid, none of the disease-causing fungi grew. In the pH 12 extract liquid, *Pythium spp*. exhibited the growth degree of 64%, *Rhizoctonia spp*. AG2-2 exhibited the growth degree of 6%, and *Sclerotinia homoeocarpa Bennett* did not grow.

### (4) Antimicrobial treatment test for cyclamen

Sixty No. 6 pots (about 18 cm in diameter) in which cyclamen was planted were prepared, and thirty pots among the sixty were randomly selected on November, and the rubber chips and/or pieces in Example 1 were sprinkled to be contacted directly with the soil surface in each pot at a rate of 5 g/pot. In that year, normal cyclamen flowers bloomed in each of the treated pots and the non-treated pots. In next April, all the flowers are removed from the sixty pots, and all the pots were managed at a place where sun light hit upon the pots through glass over a half day in every day. In the next autumn, since the non-treated pots were infected with ordinary pathogenic fungi, flowers bloomed only in four pots among the thirty non-treated ones. On the other hand, normal flowers bloomed in twenty nine pots among the thirty treated pots. This confirms that the rubber chips and/or pieces in Example 1 exhibited the antimicrobial effect.

### Example 2

Sand soil was charged into each of thirty Wagner pots (about 16 cm in inner diameter), zoysia spp. was planted in the pots, and wheat bran in which Rhizoctonia spp. was cultivated was innoculated into the pots on May 8. Raw materials having a compounding recipe shown in Table 2 were kneaded in a kneader ordinarily used in the plastic industry, and cut into cubic pieces having about 1 mm side with a true specific gravity of 2.3 by means of a hot cutter. The obtained sample was broadcasted into the pots under conditions: 0 g, 1.0 g (about 50 g/m²), 2.0 g (about 100 g/m²), 6.0 g (about 300 g/m²), 10.0 g (about 500 g/m²), and 20.0 g(about 1000 g/m²).

The thus treated pots were managed, and occurrence of the disease was checked one month after the treatment. Results are shown in Table 3.

**Table 2**

| Compounding ingredient | parts by weight |
|---|---|
| Rubber *1 | 100 |
| Barium sulfate | 580 |
| Plasticizer (process oil) *2 | 50 |
| Tolchlofos-methyl (o-2,6-dichloro-p-tolyl o,o-dimethyl phosphorothioate) powder | 30 |
| Pigment (Green LX) | 5 |

| | |
|---|---|
| *1 Low crystallinity 1,2-syndiotactic polybutadiene RB810 (manufactured by JSR) | |
| *2 Process oil PW-90 (manufactured by IDEMITSU KOUSAN Co., Ltd.) | |

**Table 3**

| Broadcasting rate (g) | Occurrence of disease (%) |
|---|---|
| 0 | 100 |
| 1.0 | 40 |
| 2.0 | 40 |
| 6.0 | 0 |
| 10.0 | 0 |
| 20.0 | 0 |

The occurrence of the disease was judged based on whether large batch occurred or not.

With respect to the pots in which the disease was not recognized (6.0 g, 10.0 g and 20.0 g), the Rhizoctonia spp. was innoculated to them on September 10, and occurrence of the disease was checked one month after the innoculation. Results are shown in Table 4.

**Table 4**

| Broadcasting rate (g) | Occurrence of disease (%) |
|---|---|
| 6.0 | 0 |
| 10.0 | 0 |
| 20.0 | 0 |

As mentioned above, according to the present invention, the occurrence of the large batch with Rhizoctonia spp. could be effectively controlled over an extended time period.

### Example 3

An asulam 80% (a 80% diluted preparation of asulam with CaCO₃) (Tradename Arjiran 80SG Arsilan) was kneaded with rubber having the compounding recipe of Table 1 at a weight ratio of 1:10, the kneaded composition was vulcanized and the vulcanizate was grounded by a grinder. The ground pieces were sieved by using a sieve, thereby obtaining the sieved pieces in a range of 1.0 to 3.0 mm. Then, the seived piece-kneaded rubber chips and/or pieces were broadcasted onto 16 zones defined by dividing a 1 m² of a turf which were used as 4 repeated grounds.

| | | | | | | | |
|---|---|---|---|---|---|---|---|
| Control | 0.0g | | 100.0g | | 200.0g | | 300.0g |
| (++) | +++++ | (-)2 | | (-)0 | | (-)1 | |
| | 100.0g | Control | 0.0g | | 200.0g | | 300.0g |
| (+)7 | + | (++++) | ++++++ | (-)3 | | (-)0 | |
| | 100.0g | | 200.0g | Control | 0.0g | | 300.0g |
| (-)0 | | (-)1 | | (+) | ++++ | (-)0 | |
| | 300.0g | | 100.0g | | 200.0g | Control | 0.0g |
| (-)0 | | (-)1 | | (-)0 | | (++) | ++++++++ |

- Broadcasting date:: May 20, 1997
- Checked date:: July 10, 1997
- Checked date:: October 10, 1997
- Name of weeds:: Cerastium glomeratum Thuill, Flat sedge(Cyperus microiria Steud.), Horseweed(Erigeron canadensis L.), Henbit(Lamium amplexicaule L.), Leibesgrasse(Eragrostis multicanlis Steud.)
"+" means that less than 10 weeds grew, "-" means that no or less than 5 weeds grew, "+" means that less than 20 weeds grew, ···. The parenthesized symbols (+), (++), (-), ··· are the results on July 20 observation, not parenthesized symbols are the results on the October 10 observation.

At the time of the observation on October 10, 1997, red rust disease harshly occurred in the control zones only, which confirmed that the antimicrobial and anti-alga agent according to the present invention exhibits sanitizing function in combination with the grass controlling function of the asulam. This test very clearly shows that the antimicrobial and anti-alga agent according to the present invention lasted the antimicrobial effect over more than 5 months because the asulam was kneaded into the rubber. It was also confirmed that since no difference in the turfgrass growth was observed between the controlled zones and the agent-broadcasted zones, the agent of the invention gave no chemical damage upon the turfgrass.

As mentioned above, when the plant antimicrobial and anti-algae particles according to the present invention are applied around various plants, the growth of the bacterial and the algae can be suppressed. Further, if the plant-growing structure and the bacterial and algae growth-controlling method according to the present invention is used, the disease damages upon the plants with the pathogenic fungi can be controlled, and the growth of the algae can be controlled.

## Claims

1. An antimicrobial and anti-algae agent for plants, said agent comprising chips and/or pieces made of rubber or plastic.

2. The antimicrobial and anti-algae agent set forth in claim 1, which contains at least one kind of an ingredient selected from the group consisting of zinc oxide, stearic acid, an activating agent, a processing aid, filler, vulcanization accelerator, resin, viscosity aid, tackifier aid, and sulfur.

3. The antimicrobial and anti-algae agent set forth in claim 1 or 2, wherein each of the chips and/or pieces has a true specific gravity of 0.5 to 4.0.

4. The antimicrobial and anti-algae agent set forth in any one of claims 1 to 3, which each of the chips and/or pieces has a volume of each of the chips and/or pieces is 0.00005-3.0 cm³.

5. A plant-growing structure comprising a plant, a cultivating medium at which said plant grows, and the antimicrobial and anti-algae agent set forth in any one of claims 1 to 4 and applied around the plant.

6. The plant-growing structure set forth in claim 5, wherein said medium is soil, and the antimicrobial and anti-algae agent is directly contacted with a surface of the soil.

7. The plant-growing structure set forth in claim 5 or 6, wherein said medium is soil, and the antimicrobial and anti-algae agent is arranged inside the soil.

8. The plant-growing structure set forth in any one of claims 5 to 7, wherein said medium is soil, a layer of water is formed on a surface of the soil, and the antimicrobial and anti-algae agent is arranged inside the water layer.

9. A method for controlling growth of fungi and algae in a plant-growing structure comprising a plant and a cultivating medium at which the plant grows, said method comprising the step of applying the antimicrobial and anti-algae agent set forth in any one of Claims 1 to 4 around the plant.
